# EUROPÄISCHE PATENTANMELDUNG

(11) **EP 1 243 229 A2**
(43) Veröffentlichungstag der Anmeldung: **25.09.2002**
(21) Anmeldenummer: 02004827.8
(22) Anmeldetag: 02.03.2002
(51) Int. Cl.: A61C 8/00

(54) **Zahnimplantat**

(30) Priorität: 23.03.2001 DE 10114627
(71) Anmelder: Dr.-medic-stom./UMF Temeschburg, Herbert Hatzlhoffer, 76337 Waldbronn (DE)
(72) Erfinder: Dr.-medic-stom./UMF Temeschburg, Herbert Hatzlhoffer, 76337 Waldbronn (DE)
(74) Vertreter: Blumenröhr, Dietrich

(57) **Zusammenfassung**

Die Erfindung betrifft ein Implantat zum Einschrauben in den Kieferknochen eines Patienten. Hierbei erstreckt sich das Schraubgewinde nur über einen ersten axialen Teilbereich des Implantats auf dessen apikaler Seite, während auf der kranialen Seite ein zweiter axialer Teilbereich vorgesehen ist, der gewindefrei ausgeführt ist und zur Abstützung des Implantats an der Kortikalis des Kieferknochens dient und hierzu einen gegenüber dem Schraubgewinde des ersten axialen Teilbereichs vergrößerten Außendurchmesser aufweist. Um sicherzustellen, dass das Schraubgewinde unterhalb des Höhenniveaus der Kortikalis angeordnet wird, weist der gewindefreie zweite axiale Teilbereich eine axiale Länge von zumindest einem Drittel der Implantatgesamtlänge auf.

## Beschreibung

Die Erfindung betrifft ein Implantat zum Inserieren in den Kieferknochen eines Patienten, mit einem an seinem äußeren Umfang vorgesehenen Schraubgewinde und mit einer an seinem kranialen Ende vorgesehenen Aufnahme zum Festlegen eines Zahnaufbaus.

Zwar gibt es zur Anpassung an verschiedene Einbauverhältnisse, beispielsweise an Kieferknochenfestigkeiten, Einbaupositionen und dergleichen, bereits eine größere Anzahl verschiedener Implantatformen; ein prinzipieller Aufbau eines Implantats besteht derzeit jedoch darin, dass sich das Schraubgewinde über fast den gesamten axialen Längenbereich erstreckt und dass sich lediglich am kranialen Ende des Implantats ein Bund mit vergrößertem Außendurchmesser anschließt, der im obersten Bereich der Kortikalis und dem Übergangsbereich zum Zahnfleisch angeordnet wird, die Aufnahme für den Zahnaufbau trägt und einen kontinuierlichen Übergang zu diesem Zahnaufbau ermöglicht. Die Stabilität eines solchen Implantats wird primär durch die Verbindung zwischen Schraubgewinde und Kieferknochen und sekundär durch das Verwachsen des Implantats mit dem Kieferknochen, durch die sogenannte Osseointegration erhalten. Demzufolge werden Implantate in der Regel in den Kieferknochen eingeschraubt und erst nach einer Einheilungszeit von ca. 3-4 Monaten mit dem Zahnaufbau versehen, durch den das Implantat erst Kräften in Längs- und Querrichtung ausgesetzt wird. Selbst wenn also ein Teil der Implantatstabilität durch Osseointegration erhalten wird, die nicht direkt von der Qualität der Verschraubung beeinflusst wird, so wird dennoch angestrebt, die Primärstabilität zu optimieren und dadurch die Voraussetzung für eine gute Osseointegration zu verbessern.

Es ist somit die Aufgabe der vorliegenden Erfindung, ein Implantat zur Verfügung zu stellen, das eine verbesserte Primärstabilität aufweist.

Diese Aufgabe wird erfindungsgemäß dadurch gelöst, dass sich das Schraubgewinde nur über einen ersten axialen Teilbereich des Implantats auf dessen apikaler Seite erstreckt, dass auf der kranialen Seite ein zweiter axialer Teilbereich vorgesehen ist, der gewindefrei ausgeführt ist und zur Abstützung des Implantats an der Kortikalis des Kieferknochens dient und hierzu einen gegenüber dem Schraubgewinde des ersten axialen Teilbereichs vergrößerten Außendurchmesser aufweist, und dass der zweite axiale Teilbereich eine axiale Länge von zumindest einem Drittel der Implantatgesamtlänge aufweist, und dass das Schraubgewinde des ersten axialen Teilbereichs einen sich verjüngenden Gewindegrund mit in Richtung des apikalen Endes abnehmendem Innendurchmesser aufweist.

Die beschriebene erfindungsgemäße Aufteilung des Implantats in einen Gewindebereich und in einen gewindefreien Bereich hat folgenden Effekt: Das Implantat wird unter Durchquerung der Kortikalis in die Spongiosa eingeschraubt und dort insbesondere zur Aufnahme von Längskräften und Drehmomenten verankert. Der gewindefreie Teilbereich beaufschlagt die Kortikalis, weitet sie radial nach außen auf und dient hierdurch zur Aufnahme von Querkräften. Insgesamt erhält man somit ein Implantat mit vorteilhafter Funktionentrennung, dessen Verankerung zur Aufnahme von Längskräften im apikalen Endbereich der Spongiosa und dessen Querkraftverankerung im - frei von Längskräften bleibenden - kranialen Bereich der Kortikalis erfolgt, wodurch sich nicht nur die Primärstabilität erhöhen, sondern auch die Osseointegration aufgrund der höheren Belastbarkeit des Implantats verbessern lässt. Demzufolge sollte der gewindefreie zweite axiale Teilbereich mindestens so lang sein wie die Kortikalis im Implantatbereich dick ist, um der Kortikalis tatsächlich eine gewindefreie großflächige Anlagefläche zur Verfügung stellen zu können. Der Restbereich der axialen Implantatlänge, der sich im Bereich der Spongiosa erstreckt, sollte zweckmäßigerweise mit dem Schraubgewinde versehen sein.

Die Verankerung in der Spongiosa wird vorteilhafterweise durch eine spezielle Gewindeform mit sich apikal verjüngendem Gewindegrund verbessert, durch die der weiche Knochen der Spongiosa entlang des Schraubgewindes beim Inserieren langsam schrittweise komprimiert wird. In ähnlicher Weise arbeitet der gewindefreie zweite axiale Teilbereich, dessen Außendurchmesser gegenüber dem Schraubgewinde vergrößert ist und hierdurch für eine weitere Komprimierung des Knochens und damit für einen besseren Halt sorgt.

Für die Schraubverankerung des Implantats in der Spongiosa ist es besonders zweckmäßig, wenn das Schraubgewinde einen zumindest in etwa gleichbleibenden Außendurchmesser aufweist, so dass die Gewindetiefe zwischen Außendurchmesser des Schraubgewindes und dem sich verjüngenden Gewindegrund in Apikalrichtung stetig zunimmt. Hierbei schneidet der Gewindegang des Schraubgewindes zwar gleich zu Beginn den im gesamten Schraubgewindebereich wirksamen Außendurchmesser in den Kieferknochen ein, der Bereich des Gewindegrundes verdrängt aber in der oben beschriebenen Art und Weise ausgehend vom apikalen Ende aufgrund seines sich stetig vergrößernden Innendurchmessers nur schrittweise den Kieferknochen und vergrößert somit die Kompression und damit die Verankerung des Kieferknochens behutsam, aber stetig.

Vorteilhafterweise ist das Schraubgewinde als Trapezgewinde ausgebildet, wodurch man durch den breiten Gewindegang eine größere Verdrängung des Kieferknochens und einen besseren Halt des Schraubgewindes im Kieferknochen erhält, wobei Trapezgewinde darüber hinaus tatsächlich primär dazu vorgesehen sind, Längskräfte zu übertragen.

Für das erwähnte schrittweise Komprimieren des Kieferknochens durch das Schraubgewinde ist es von Vorteil, wenn der sich verjüngende Gewindegrund entlang einer etwa kegelstumpfförmigen Mantelfläche verläuft, die einen Kegelneigungswinkel α gegenüber der Schraubachse von etwa 5° aufweist.

Ein schonendes Inserieren des Implantats lässt sich dadurch erzielen, dass das Schraubgewinde zweigängig ausgebildet ist. Dieses zweigängige Schraubgewinde lässt sich zur Erzielung einer vergrößerten Verdrängungsfläche dadurch ausnutzen, dass ausgehend von dem in etwa gleichbleibenden Außendurchmesser die Gewindetiefe des zweiten Gewindegangs kleiner ausgebildet ist als die Gewindetiefe des ersten Gewindegangs, wodurch der Gewindegrund des zweiten Gewindegangs eine geringere Axialerstreckung aufweist und die axiale Länge der außenliegenden Gewindegänge vergrößert werden kann. Als Gewindetiefe des zweiten Gewindegangs empfiehlt sich etwa die halbe Gewindetiefe des ersten Gewindegangs im jeweiligen Axialabschnitt, wodurch der Kegelneigungswinkel β der ebenso etwa kegelstumpfförmigen Mantelfläche des zweiten Gewindegangs etwa 2° bis 5° beträgt, insbesondere aber mit einem halb so großen Kegelneigungswinkel gegenüber der Schraubachse von etwa 2,5° verläuft.

Das zweigängige Schraubgewinde hat außerdem den Vorteil, dass das Implantat beim Einschrauben pro Umdrehung gleich zwei Gewindegänge in Wirkverbindung mit der Spongiosa bringt. So reichen schon wenige Umdrehungen (in einem Ausführungsbeispiel gerade einmal etwas mehr als zwei) für ein dauerhaftes, sicheres und gebrauchsfertiges Inserieren aus.

Für den Unterkieferfrontzahnbereich, der sich durch eine sehr hohe Knochendichte auszeichnet, darf das Schraubgewinde des Implantats den Knochen nicht in zu großem Maße komprimieren, um eine Beschädigung des Kieferknochens durch Rissbildungen oder sogar durch ein Aufsprengen zu verhindern; deshalb empfiehlt sich anstelle eines Doppelgewindes mit gleichbleibendem Außendurchmesser die Verwendung eines einfachen Schraubgewindes, dessen Außendurchmesser in gleicher Weise wie der Gewindegrund unter einem Kegelneigungswinkel 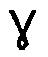 von 5° zur Schraubachse verläuft. Zur weiteren Reduzierung der Komprimierungseigenschaften weist das Schraubgewinde eine vergleichsweise große Steigung auf, aber dennoch flach geneigte Gewindeflanken.

Was den zweiten axialen Teilbereich betrifft, der gewindefrei ausgeführt ist und sich im Bereich der Kortikalis erstreckt, so empfiehlt sich auch für diesen eine etwa konische lichte Außenform, die sich in apikaler Richtung verjüngt, um auch in diesem axialen Teilbereich eine langsam ansteigende Komprimierung des Kieferknochens und somit eine verbesserte Verankerung in der Kortikalis zu erhalten. Zweckmäßigerweise ist der zweite axiale Teilbereich glattwandig ausgeführt. Ebenso ist es aber auch möglich, ihn profiliert mit umlaufenden ringförmigen Querschnittsreduzierungen auszuführen, die im Axialschnitt gesehen eine kreisbogenförmige Außenform zwischen sich belassen. Ebenso ist natürlich auch eine Mischform zwischen glattwandiger und profilierter Gestaltung der äußeren Mantelfläche des zweiten axialen Teilbereichs möglich.

Zweckmäßigerweise besteht der zweite axiale Teilbereich aus einem rohrförmigen Aufsatz, der separat vom ersten axialen Teilbereich gestaltet ist, so dass zunächst einmal das Implantat in den Kieferknochen eingeschraubt werden kann und erst anschließend der zweite axiale Teilbereich auf den eingeschraubten ersten axialen Teilbereich aufgesetzt und an der Kortikalis festgelegt wird. In diesem Zusammenhang ist es von Vorteil, wenn der erste axiale Teilbereich einen kranialen Fortsatz mit gegenüber dem Schraubgewinde reduzierten Außendurchmesser aufweist, und wenn auf den Fortsatz der den zweiten axialen Teilbereich bildende rohrförmige Aufsatz aufgesteckt ist. Das Festlegen des den zweiten axialen Teilbereich bildenden rohrförmigen Körpers auf den Fortsatz kann über Schraubmittel und insbesondere über Festlegungsmittel für den Zahnaufbau erfolgen, insbesondere wenn der Fortsatz die Aufnahme für den Zahnaufbau trägt.

Die separate Gestaltung des zweiten axialen Teilbereichs als rohrförmiger Aufsatz und getrennt vom ersten axialen Teilbereich hat den besonderen Vorteil, dass bei einer Erkrankung des Implantatbetts (sog. Implantitis) dieser Aufsatz ohne Schwierigkeiten ausgetauscht werden kann. Hierbei kann man nicht nur einen infizierten Aufsatz durch einen neuen sterilen Aufsatz ersetzen, sondern vor allem bietet das Entfernen des Aufsatzes die Möglichkeit, ohne große Schwierigkeiten an die infizierten Knochenbereiche zu gelangen. Während bisher bei derartigen Erkrankungen das Implantat entfernt werden musste und der Inserierungs- und Einheilungsprozess nach der Behandlung wieder von vorne beginnen musste, kann nun das Implantat in der inserierten und osseointegrierten Stellung verbleiben; es wird lediglich der Aufsatz entfernt, die infizierten Knochenbereiche behandelt bzw. abgetragen, ein neuer steriler Aufsatz wieder aufgesetzt und anschließend wieder der Knochenaufbau im behandelten Bereich initialisiert, ohne dass die benachbarten Knochenbereiche - wie es beim Stand der Technik der Fall ist - nach einem Neuimplantieren ebenfalls bearbeitet worden sind und erneut aufgebaut werden müssen.

Was den weiteren Aufbau des Implantats betrifft, so weist dieses insbesondere im Axialbereich des Schraubgewindes ein Innengewinde auf, in dem entweder während der 3-4-monatigen Zeit der Osseointegration eine Einheilschraube oder ein Gingivaformer und/oder anschließend Befestigungsschrauben für den Zahnaufbau verankert werden, falls keine sofortige Behandlung des Implantats erfolgt .

Schließlich sei noch erwähnt, dass das Implantat insbesondere im Bereich des Schraubgewindes eine chemisch aufgeraute Oberfläche aufweisen kann. Hingegen können Teile des zweiten axialen Teilbereichs mit glatter Oberfläche und ohne Aufrauung gestaltet sein.

Weitere Merkmale und Vorteile der vorliegenden Erfindung ergeben sich aus der nachfolgenden Beschreibung von Ausführungsbeispielen anhand der Zeichnung; hierbei zeigen
- Figur 1: das erfindungsgemäße Implantat eingesetzt in einen Kieferknochen in geschnittener Seitenansicht;
- Figur 2: einen ersten, das Schraubgewinde aufweisenden axialen Teilbereich mit kranialem axialem Fortsatz im Längsschnitt;
- Figur 2a: ein Detail des Schraubgewindes aus Figur 2;
- Figur 3: eine alternative Ausführungsform eines Implantats in geschnittener Seitenansicht;
- Figuren 4-6: unterschiedliche Ausführungsbeispiele für rohrförmige Aufsätze, wel-che den gewindefreien zweiten axialen Teilbereich des Implantates bilden.

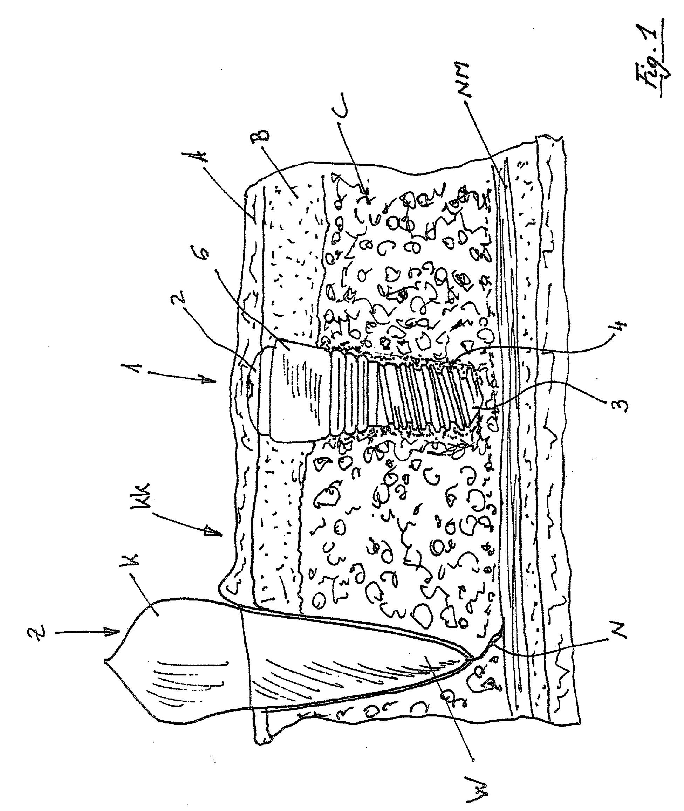

In Figur 1 ist ein Kieferknochen in einem schematischen Vertikalschnitt dargestellt, wobei der Aufbau des Kieferknochens KK gezeigt ist und die Orientierung eines Zahnes Z sowie eines eingesetzten Implantats zu den einzelnen Knochenbereichen: Die äußerste Schicht bildet das Zahnfleisch A, das an der Krone K des Zahnes Z dicht anliegt und das Implantat 1 während der in Figur 1 dargestellten Einheilphase im Bereich einer Einheilschraube 2 überdeckt. Die nächste Schicht ist die Kortikalis B, die für den Halt des Zahns bzw. des Implantats vor allem in Querrichtung sorgt. Dem schließt sich die Spongiosa C an, die porös aufgebaut ist und den großflächigsten Knochenbereich bildet. In der Spongiosa C ist der Zahn Z mit seiner Zahnwurzel W verankert, von wo aus sich der Zahnnerv N bis zum nervus mandibularis NM des Kieferknochens KK erstreckt.

Das Implantat 1 ist in ähnlicher Weise wie der dargestellte Zahn Z im Kieferknochen angeordnet und weist einen der Zahnwurzel entsprechenden ersten axialen Teilbereich 3 auf, der ein Schraubgewinde 4 auf seiner Außenseite trägt und im Bereich der Spongiosa angeordnet ist und hierdurch das apikale Ende des Implantats bildet. Ein zweiter axialer Teilbereich 5 ist gewindefrei ausgeführt und erstreckt sich auf dem Höhenniveau der Kortikalis und dient dazu, das Implantat in Querrichtung an der Kortikalis abzustützen, ohne es durch Gewindegänge längsgerichteten Kraftkomponenten auszusetzen. Würde sich statt dessen das Schraubgewinde bis in den Bereich der Kortikalis erstrecken, so würden in Axialrichtung wirkende Längskraftkomponenten zu einer Beanspruchung des Implantatsitzes im kritischen und für den Halt verantwortlichen Kortikalisbereich führen; trotz einer guten Osseointegration am Ende der Einheilzeit führt dies jedoch leicht dazu, dass der Sitz des Implantats in der Kortikalis bei stärkeren Belastungen gelockert wird.

Im Gegensatz dazu schlägt nun die vorliegende Erfindung vor, den Bereich der Kortikalis frei von Gewindegängen zu halten und statt dessen den dortigen zweiten axialen Teilbereich mit einem größeren Außendurchmesser zu versehen als den ersten axialen Teilbereich mit Schraubgewinde, um so ein gleichmäßiges, behutsames und großflächiges Komprimieren der Kortikalis im Umfangsbereich des Implantats zu erhalten. Gleichzeitig sorgt das auf den Bereich der Spongiosa beschränkte Schraubgewinde für die gewünschte Fixierung des Implantats in Längsrichtung.

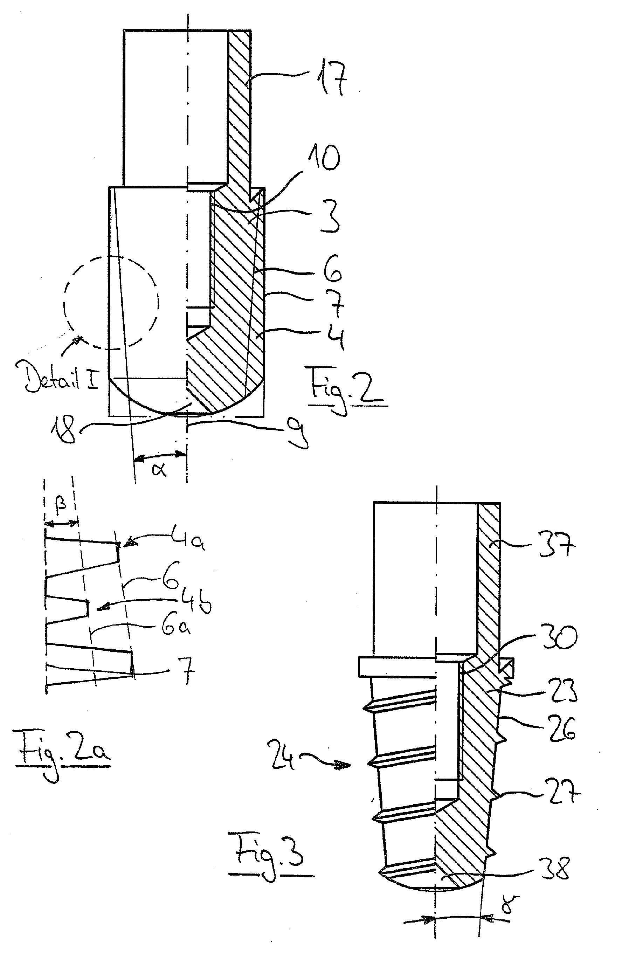

Um diesen Verankerungsbereich des Schraubgewindes 4 trotz geringerer Axiallänge verglichen mit dem Stand der Technik zu verbessern, weist das erfindungsgemäße Implantat einen sich verjüngenden Gewindegrund 6 (siehe Figur 2) mit in Richtung des apikalen Endes abnehmendem Innendurchmesser auf, während der Außendurchmesser des Schraubgewindes 4 im wesentlichen gleich bleibt. Hierdurch erhält man ebenfalls ein behutsames Komprimieren der Spongiosa im Bereich des Gewindegrundes, der beim Einschrauben des Implantats eine kontinuierliche radiale Aufweitung erzeugt.

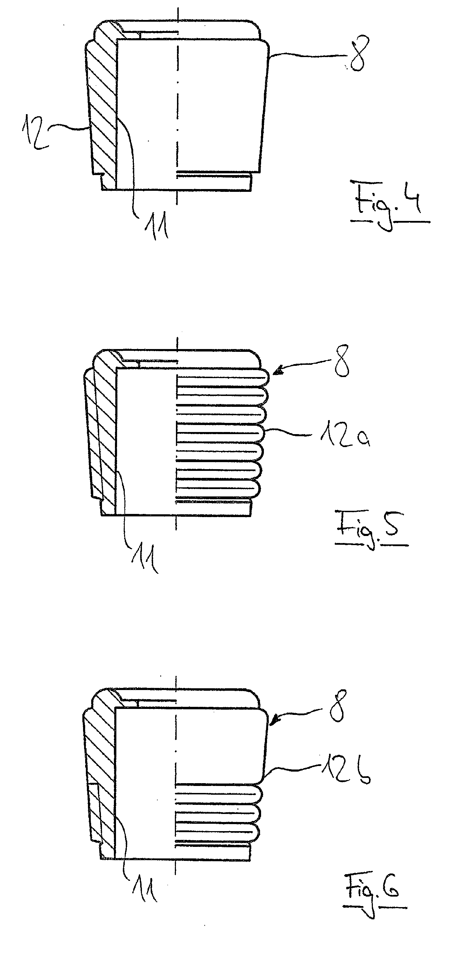

Dieser erste axiale Teilbereich 3 mit Schraubgewinde 4 ist in Figur 2 im Detail dargestellt, wobei zu erkennen ist, dass er in kranialer Axialrichtung einen Fortsatz 17 trägt, der zur Festlegung des zweiten gewindefreien axialen Teilbereichs 5 dient, der als rohrförmiger Körper 8 ausgebildet ist und in verschiedenen Ausführungsformen in den Figuren 4 bis 6 dargestellt ist.

Das in Figur 2 gezeigte Schraubgewinde 4 weist folglich einen sich verjüngenden Gewindegrund 6 auf, der entlang einer etwa kegelstumpfförmigen Mantelfläche verläuft, deren Kegelneigungswinkel α gegenüber der Schraubachse 9 etwa 5° beträgt. Aus Figur 2 ist auch der etwa gleichbleibende Außendurchmesser 7 des Schraubgewindes zu erkennen, so dass die Gewindetiefe in Apikalrichtung langsam zunimmt. Schließlich zeigt Figur 2 im apikalen Endbereich eine prismenförmige und nicht rotationssymmetrische Aussparung 18, die sich quer zur Schraubachse erstreckt und eine Verdrehsicherung gegen unbeabsichtigtes Lösen bzw. Herausdrehen des osseointregrierten Implantats bildet.

Ein Detail I aus Figur 2 ist in dem Ausschnitt in Figur 2a gezeigt: Dort ist zu erkennen, dass das Schraubgewinde 4 zweigängig ausgeführt ist mit einem ersten Gewindegang 4a und einem zweiten Gewindegang 4b, wobei die Gewindetiefe des ersten Gewindegangs größer und insbesondere ― bezogen auf den jeweiligen Axialbereich ― doppelt so groß wie die Gewindetiefe des zweiten Gewindegangs 4b bis zum Gewindegrund 6a ausgebildet ist. Außerdem ist das Schraubgewinde 4 als Trapezgewinde ausgeführt, so dass sich insgesamt die außenliegenden Gewindegangbereiche in Axialrichtung vergrößern lassen, was zu einer Vergrößerung der vom Implantat verdrängten Knochenbereiche und somit zu einem besseren Halt des Implantats in der Spongiosa führt.

Im vorliegenden Ausführungsbeispiel ist der erste axiale Teilbereich 3 etwa 60 µm lang und der Außendurchmesser 40 µm groß und die Gewindetiefe im apikalen Anfangsbereich beim ersten Gewindegang beträgt 5 µm und beim zweiten Gewindegang 2,5 µm. Die Steigung der beiden Gewindegänge beträgt 7 µm und die Gewindeflanken weisen eine Neigung zur Schraubachse von ca. 75° auf.

Auf der radialen Innenseite des ersten axialen Teilbereichs 3 ist ein Schraubinnengewinde 10 vorgesehen, das zur Festlegung von Aufbauten, prothetischen Hilfsteilen etc. dient.

Der kraniale Fortsatz 17 ist rohrförmig ausgebildet mit kreisförmigem Querschnitt und nimmt den erwähnten rohrförmigen Körper 8 auf, der den zweiten axialen Teilbereich bildet, der später näher beschrieben wird.

Figur 3 zeigt eine alternative Ausführungsform eines ersten axialen Teilbereichs 23 eines Implantats 21 mit einem Schraubgewinde 24, welches eingängig ausgeführt ist mit einer vergleichsweise großen Gewindesteigung (im gezeigten Ausführungsbeispiel von P = 14 µm), einem sich in Apikalrichtung verjüngenden Gewindegrund 26, der entlang einer etwa kegelstumpfförmigen Mantelfläche verläuft, deren Kegelneigungswinkel 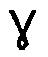 gegenüber der Schraubachse 29 etwa 5° beträgt. Im Gegensatz zum Ausführungsbeispiel aus Figur 2 ist auch der Außendurchmesser 27 des Schraubgewindes 24 sich in Apikalrichtung verjüngend ausgebildet, so dass insgesamt die Kompression des vorliegenden Schraubgewindes weitaus geringer ausfällt als im ersten Ausführungsbeispiel. Hierdurch ist dieses Implantat 21 für die hochdichten Frontzahnbereiche im Unterkiefer geeignet, die bei zu großer Kompression reißen bzw. aufgesprengt werden könnten.

Im übrigen entsprechen Fortsatz 37, Aussparung 38 und Innengewinde 30 den entsprechenden Bauteilen 17, 18 und 10 aus Figur 2 in Aufbau und Funktion.

Der in Figur 4 dargestellte rohrförmige Aufsatz 8 weist entsprechend dem Fortsatz 17 bzw. 37 eine zylindrische Innenbohrung 11 auf, über die er großflächig und spielfrei an dem Fortsatz 17 anliegt, um direkt die wirksamen Kräfte ein- bzw. ausleiten zu können. Die Außenseite 12 des Aufsatzes 8 ist konisch ausgebildet mit sich in kranialer Axialrichtung vergrößerndem Außendurchmesser. Hierdurch wird beim Einsetzen dieses Aufsatzes die angrenzende Kortikalis schrittweise komprimiert und die entsprechende Flächenpressung sukzessive erhöht, bis der Aufsatz keilähnlich in der Kortikalis verankert ist, was vor allem für eine Seitenstabilität des Implantats sorgt.

Was die Außenform dieser Außenseite 12 betrifft, so sind ausgehend von einer glattwandigen Konusform auch verschiedene Profilierungen denkbar: Beispielsweise in Figur 5 ist eine über den gesamten Axialbereich sich erstreckende Profilierung 12a aus umlaufenden ringförmigen Querschnittsreduzierungen gebildet, die im Längsschnitt eine aus aneinander gefügten kreisbogenförmigen Abschnitten bestehende Außenprofilierung bilden. Hierdurch lässt sich die Oberfläche gegenüber einer glattwandigen Außenseite noch einmal vergrößern und der Anlagebereich des Implantats an der Kortikalis entsprechend optimieren. Darüber hinaus ist natürlich auch eine Mischform 12b entsprechend Figur 6 möglich, wo der obere Aufsatzbereich glattwandig und der unter Aufsatzbereich profiliert ausgeführt ist.

Besonders wesentlich ist die axiale Länge des rohrförmigen Aufsatzes 8: Damit sich das Gewinde des eingeschraubten Implantats nicht bis in den Bereich der Kortikalis erstreckt, sollte dieser zweite axiale und gewindefreie Teilbereich diesen Höhenabschnitt der Kortikalis vollständig ausfüllen und hierzu empfiehlt es sich, dass der zweite axiale Teilbereich eine axiale Länge von zumindest einem Drittel der Implantatgesamtlänge aufweist.

Zusammenfassend bietet die vorliegende Erfindung den Vorteil eines Implantats mit optimierter Verankerung im Kieferknochen, wobei erstmals ein Implantat mit Funktionentrennung dadurch zur Verfügung gestellt wird, dass sich das Schraubgewinde nur auf den Bereich der Spongiosa erstreckt und zur Aufnahme von Längskräften dient, während der gewindefreie zweite axiale Teilbereich auf dem Höhenniveau der Kortikalis angeordnet ist und Querkraftkomponenten überträgt.

## Patentansprüche

1. Implantat zum Einschrauben in den Kieferknochen (KK) eines Patienten, mit einem an seinem äußeren Umfang vorgesehenen Schraubgewinde (4, 24) und mit einer an seinem kranialen Ende vorgesehenen Aufnahme zum Festlegen eines Aufbaus, **dadurch gekennzeichnet,**
**dass** sich das Schraubgewinde (4, 24) nur über einen ersten axialen Teilbereich (3, 23) des Implantats (1, 21) auf dessen apikaler Seite erstreckt, dass auf der kranialen Seite ein zweiter axialer Teilbereich (5) vorgesehen ist, der gewindefrei ausgeführt ist und zur Abstützung des Implantats an der Kortikalis des Kieferknochens dient und hierzu einen gegenüber dem Schraubgewinde des ersten axialen Teilbereichs vergrößerten Außendurchmesser aufweist, und dass der zweite axiale Teilbereich eine axiale Länge von zumindest einem Drittel der Implantatgesamtlänge aufweist.

2. Implantat nach Anspruch 1,
**dadurch gekennzeichnet,**
**dass** das Schraubgewinde (4, 24) des ersten axialen Teilbereichs (3, 23) einen sich verjüngenden Gewindegrund (6, 26) mit in Richtung des apikalen Endes abnehmendem Innendurchmesser aufweist.

3. Implantat nach zumindest einem der vorstehenden Ansprüche,
**dadurch gekennzeichnet,**
**dass** das Schraubgewinde (4) einen zumindest in etwa gleichbleibenden Außendurchmesser (7) aufweist.

4. Implantat nach zumindest Anspruch 2,
**dadurch gekennzeichnet,**
**dass** der sich verjüngende Gewindegrund (6, 26) entlang einer etwa kegelstumpfförmigen Mantelfläche verläuft mit einem Kegelneigungswinkel α bzw. 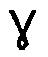 gegenüber der Schraubachse (9, 29) von etwa 5°.

5. Implantat nach zumindest einem der vorstehenden Ansprüche,
**dadurch gekennzeichnet,**
**dass** das Schraubgewinde (4) zweigängig ausgebildet ist.

6. Implantat nach zumindest Anspruch 3 und Anspruch 5,
**dadurch gekennzeichnet,**
**dass** ausgehend von dem in etwa gleichbleibenden Außendurchmesser (7) die Gewindetiefe des zweiten Gewindegangs (4b) kleiner ausgebildet ist als die Gewindetiefe des ersten Gewindegangs (4a).

7. Implantat nach Anspruch 6,
**dadurch gekennzeichnet,**
**dass** der Gewindegrund (6a) des zweiten Gewindegangs entlang einer etwa kegelstumpfförmigen Mantelfläche verläuft mit einem Kegelneigungswinkel β gegenüber der Schraubachse von zwischen 2 und 5°.

8. Implantat nach zumindest einem der vorstehenden Ansprüche,
**dadurch gekennzeichnet,**
**dass** das Schraubgewinde (4) als Trapezgewinde ausgebildet ist.

9. Implantat nach zumindest einem der vorstehenden Ansprüche,
**dadurch gekennzeichnet,**
**dass** der zweite axiale Teilbereich (5) eine etwa konische lichte Außenform aufweist, die sich in apikaler Richtung verjüngt.

10. Implantat nach zumindest Anspruch 9,
**dadurch gekennzeichnet,**
**dass** der zweite axiale Teilbereich (5) glattwandig ausgeführt ist.

11. Implantat nach Anspruch 9,
**dadurch gekennzeichnet,**
**dass** der zweite axiale Teilbereich (5) profiliert ausgeführt ist.

12. Implantat nach zumindest Anspruch 11,
**dadurch gekennzeichnet,**
**dass** die Profilierung aus umlaufenden ringförmigen Querschnittsreduzierungen unter Belassung von im Axialschnitt gesehenen kreisbogenförmigen Außenprofilierungen besteht.

13. Implantat nach zumindest einem der vorstehenden Ansprüche,
**dadurch gekennzeichnet,**
**dass** der zweite axiale Teilbereich (5) aus einem rohrförmigen Aufsatz (8) besteht.

14. Implantat nach zumindest Anspruch 13,
**dadurch gekennzeichnet,**
**dass** der erste axiale Teilbereich (3, 23) einen kranialen Fortsatz (17, 37) mit gegenüber dem Schraubgewinde (4, 24) reduzierten Außendurchmesser aufweist, und dass auf den Fortsatz der den zweiten axialen Teilbereich (5) bildende rohrförmige Aufsatz (8) aufgesetzt ist.

15. Implantat nach Anspruch 14,
**dadurch gekennzeichnet,**
**dass** der Fortsatz (17, 37) die Aufnahme für den Zahnaufbau trägt.

16. Implantat nach Anspruch 14,
**dadurch gekennzeichnet,**
**dass** das Festlegen des den zweiten axialen Teilbereich (5) rohrförmigen Aufsatzes (8) auf dem Fortsatz (17) über Schraubmittel und insbesondere über Festlegungsmittel für den Zahnaufbau erfolgt.

17. Implantat nach zumindest einem der vorstehenden Ansprüche,
**dadurch gekennzeichnet,**
**dass** der erste axiale Teilbereich (3, 23) an seinem apikalen Ende eine Verdrehsicherung in Form einer nicht rotationssymmetrischen Aussparung (18, 38) aufweist.
